# EUROPEAN PATENT SPECIFICATION

(11) **EP 2 373 226 B1**
(45) Date of publication and mention of the grant of the patent: **27.03.2013**
(21) Application number: 09748259.0
(22) Date of filing: 06.11.2009
(51) Int. Cl.: A61B 17/06, A61B 17/00

(54) **SURGICAL THREAD WITH SHEATH-CORE CONSTRUCTION**
CHIRURGISCHER FADEN MIT HÜLLEN-KERN-KONSTRUKTION
FIL CHIRURGICAL À ÂME GAINÉE

(30) Priority: 06.11.2008 DE 102008057216
(43) Date of publication of application: 12.10.2011
(73) Proprietor: ITV Denkendorf Produktservice GmbH, 73770 Denkendorf (DE); Aesculap AG, 78532 Tuttlingen/Donau (DE)
(72) Inventor: ODERMATT, Erich, CH-8200 Schaffhausen (CH); BERNDT, Ingo, 52066 Aachen (DE); KÖNIG, Silke, 78628 Rottweil (DE); MÜLLER, Erhard, 70565 Stuttgart (DE); OBERHOFFNER, Sven, 71384 Weinstadt-Endersbach (DE); PLANCL, Heinrich, 72622 Nürtingen (DE)
(74) Representative: Patentanwälte Ruff, Wilhelm, Beier, Dauster & Partner
(86) International application number: PCT/EP2009/007948
(87) International publication number: WO 2010/052005

(56) References cited:
- EP-A1- 1 348 449
- WO-A2-2009/105663
- WO-A2-2009/132284
- US-A1- 2007 005 110
- US-A1- 2008 046 094
- US-A1- 2008 221 618

## Description

The present invention relates to a surgical thread having a sheath-core construction and having barbs for anchoring in biological, in particular human and/or animal, tissues, to a surgical kit and also to a method of forming the surgical thread.

Wounds are typically closed in surgery using thread-shaped sutures. These are usually knotted to achieve a secure hold in the tissue. Here care must be taken to ensure that the wounds to be closed are always stitched together using an optimal force at the wound edges. If, for example, the wound edges are stitched together too loosely and too non-uniformly, there is a risk in principle of increased scarring or of dehiscence. If, by contrast, the wound edges are stitched together overly tautly, there is a risk that blood flow through the wound edges is restricted, which can give rise to necrotic changes in the surrounding tissue region.

In addition to the risk of possible secondary complications, which may necessitate renewed surgical interventions, there is also always a certain risk that wound closures based on knotted suture will lead to disruptions in the healing process and to unsatisfactory cosmesis for the patients concerned. Another factor is that it is often necessary for several knots, in particular up to 7 knots, to be placed on top of each other to ensure a secure knotted hold. This means that there is a lot of material being introduced into the region of the wound to be cared for, and can more particularly lead to increased foreign-body reactions, particularly in the case of absorbable suture.

Barbed sutures, which unlike the familiar/conventional threads do not have to be knotted, have also been around for some time. Such knotless or self-retaining sutures usually consist of a monofil thread equipped with barbs along its longitudinal axis. Barbed sutures are described for example in US 3,123,077 A, EP 1 559 266 B1. EP 1 560 683 B1 and EP 1 555 946 B1. The barbs are configured on a thread such that the thread can be pulled through the tissue in the direction of the barbs without great resistance and without tissue trauma. When pulled in the opposite direction, however, the barbs deploy and anchor themselves and hence also the suture in the surrounding tissue region. This prevents the suture being pulled back through the puncture channel.

The barbs are produced by cutting into a drawn thread material. A problem with this is that, as a result of its having been drawn, the thread material has a narrowed diameter, so that when barbs are cut into such a thread material problems can arise with regard to mechanical strength when the cut into the thread is inaccurate and goes too deep. When the barbs are cut too deeply into the suture, even very small loads can lead to tearing and propagation of the cut sites and hence to a destabilization of the suture. Breakages in the suture can occur in extreme cases.

A co-extruded tissue grasping monofilament is known from US 2008/0221618 A1.

A linear tension material for plastic surgery comprising a core material and a textile covering material is known from US 2008/0046094 A1.

The present invention therefore has for its object to provide a surgical thread which is alternative to the knotless sutures known from the prior art and which avoids the disadvantages known from the prior art, more particularly provides adequate security and strength in relation to wound closure. The present invention further has for its object to provide a method of forming the surgical thread that has distinct processing advantages over the conventional methods of forming knotless sutures.

This object is achieved by a surgical thread having the features of independent claim 1. Preferred embodiments of the invention thread are subject matter of dependent claims 2 to 12. The present invention also provides a surgical kit having the features as per claim 13. The present invention further comprises a method of forming the surgical thread as per independent claim 14. Preferred embodiments are subject matter of dependent claims 15 to 17.

The thread of the present invention comprises a surgical thread having a polymeric core and a polymeric sheath surrounding the core (sheath-core construction), wherein the sheath includes barbs for anchoring in biological, in particular human and/or animal, tissues.

In other words, the present invention provides a surgical thread, preferably as surgical suture, having a polymeric sheath-core construction, the sheath of which includes barbs. The barbs are generally formed by cuts into the sheath.

According to the present invention, the manner in which the polymeric sheath of the thread surrounds its polymeric core may be partial only. Preferably, however, the entire area of the polymeric core is surrounded by the polymeric sheath.

In principle, the polymeric core of the thread and the polymeric sheath may be connected by covalent and/or on-covalent bonds. However, a connection of the polymeric core and the polymeric sheath by non-covalent bonds is preferred.

Furthermore, the polymeric sheath may have a textile or non-textile structure. Preferably, the polymeric sheath has a non-textile structure. In other words, the polymeric sheath is a non-textile sheath in preferred embodiments.

In a further embodiment, the polymeric sheath as such is surrounded by a tubular (hose type) construction, in particular a tubular grid and/or a tubular textile fabric, preferred is a tubular knitted fabric. Thus, there is a beneficial improvement of the linear breaking strength of the thread according to the invention. The tubular construction is preferably a netting and/or a mesh (tubular meshwork). Preferably, the barbs protrude from meshes of the tubular construction. To that end, the barbs may be cut into the thread prior to drawing, for example. A subsequent drawing of the thread will result in an erection of the barbs to make the barbs stick out from the thread in the vicinity of the meshes of the tubular construction.

In another embodiment, the polymeric sheath has a multilayer, in particular a two-layer, three-layer or four-layer etc. structure. Individual layers of the sheath may be formed from different materials. Thus, different mechanical characteristics of the sheath and thus of the thread as well may be realized. In particular, a tubular construction may be a component of a multilayer structure of the polymeric sheath, as described above for example.

In one preferred embodiment, the barbs have a barb cut depth (measured perpendicularly from the thread surface) equal to not more than the thickness of the polymeric sheath. More particularly, the barb cut depth may be exactly equal to the thickness of the polymeric sheath. The barbs preferably have a barb cut depth between 10 and 35%, in particular 15 and 30%, of the diameter of the surgical thread.

The polymeric core of the thread is preferably configured to be flexible, more particularly to be flexurally slack. It is particularly preferable according to the present invention when the polymeric core is configured to be more flexible, in particular to be flexurally slacker, than the polymeric sheath. The sheath, by contrast, is preferably configured to be stiff, in particular to be flexurally stiff. Preferably, the polymeric sheath is configured to be stiffer, in particular flexurally stiffer, than the polymeric core. This makes it possible for the thread of the present invention to have flexible properties on the one hand and stiff barbs on the other. Increased flexibility, in Particular flexural slackness, on the part of the thread is particularly advantageous in improving its handling, whereas stiff barbs generally lead to stronger anchoring of the thread in a biological tissue.

In a further embodiment, the polymeric core has a lower flexural modulus than the polymeric sheath. Preferably, the polymeric core has a flexural modulus between 200 and 2000 N/mm², preferably 300 and 1200 N/mm². The flexural modulus of the polymeric sheath is preferably between 1000 and 10 000 N/mm², in particular 1500 and 5000 N/mm².

The surgical thread can in principle be formed of any polymeric material suitable for forming sutures. The contemplated polymeric materials may comprise absorbable and/or nonabsorbable polymeric materials. In other words, it is within the scope of the invention that a partially absorbable thread may be provided. According to the present invention, the polymeric core may be formed of a nonabsorbable polymeric material and the polymeric sheath of an absorbable polymeric material, or vice versa, for example. The polymers may in particular be present as homo-, co-, ter- or tetrapolymer etc. Suitable polymeric materials are for example block polymers, in particular block co- or terpolymers. The use of random or alternating co-, ter- or tetrapolymers etc. is similarly possible according to the present invention.

Useful absorbable polymeric materials include, in particular, polymers from the group consisting of polylactide, polyglycolide, poly(ε-caprolactone), poly(para-dioxanone), poly(trimethylene carbonate), poly(hydroxybutyric acid), mixtures thereof, copolymers thereof and terpolymers thereof. Preference is given to co- or terpolymers, preferably block co- or terpolymers, that include at least one monomer from the group consisting of lactide, glycolide, trimethylene carbonate, para-diox-anone, ε-caprolactone and hydroxybutyric acid, in particular γ-hydroxybutyric acid.

Examples of suitable nonabsorbable polymeric materials are polymers from the group consisting polyolefins, polyesters, polyamides, polyurethanes, nylon, silk, cotton, mixtures thereof, copolymers thereof and terpolymers thereof. Polypropylene in particular must be mentioned as suitable polyolefin. Polyethylene terephthalate is an example of a suitable polyester.

The polymeric core of the thread has a higher melting point, preferably an at least 20°C higher melting point, than the polymeric sheath. This makes it possible, for example, to cut barbs into the thread under thermal conditions without cutting into the polymeric core of the thread. This preserves properties of the thread core, for example linear tensile strength, flexibility and/or elongation at break.

The polymeric core of the thread is preferably formed of an absorbable polymeric material, preferably having a glass transition point < 30°C. The polymeric core of the thread can be formed in particular of a co- or terpolymer, preferably block co- or terpolymer, comprising at least one monomer from the group consisting of glycolide, lactide, ε-caprolactone, trimethylene carbonate and hydroxybutyric acid. Particular preference is given to Monosyn®, which comprises a triblock terpolymer of glycolide, trimethylene carbonate and ε-caprolactone.

In an alternative embodiment, the polymeric core of the thread is formed of a nonabsorbable polymeric material. The nonabsorbable polymeric material may comprise a polymeric material from the group consisting of polyolefins, polyesters, polyamides, mixtures thereof, copolymers thereof and terpolymers thereof. The polymeric material can be selected for example from the group consisting polypropylene, polyethylene terephthalate, nylon, silk, cotton, mixtures thereof, copolymers thereof and terpolymers thereof.

The polymeric sheath is preferably formed of an absorbable polymeric material, in particular from the group consisting of poly(para-dioxanone), poly(ε-caprolactone), polyglycolide, polylactide, poly(trimethylene carbonate), poly(hydroxybutyric acid), mixtures thereof, copolymers thereof and terpolymers thereof. In an alternative embodiment, the polymeric sheath is formed of a nonabsorbable polymeric material, in particular from the group consisting of polyolefins, polyesters, polyamides, mixtures thereof, copolymers thereof and terpolymers thereof. Examples of nonabsorbable polymeric materials include polypropylene, polyethylene terephthalate, nylon, silk, cotton, mixtures thereof, copolymers thereof and terpolymers thereof.

As mentioned above, the thread of the present invention may in principle be formed of nonabsorbable polymeric materials only. For example, the polymeric core of the thread may be formed of a polyolefin, in particular polypropylene, and the polymeric sheath of polyethylene terephthalate or polyamide.

The surgical thread, in particular the polymeric core of the thread and/or the polymeric sheath, may be provided with colourings. Preferably, the polymeric core and the polymeric sheath comprise a different colour. Thus, a colour shade tigering of the thread may be realized, for example. Overall, a different dyeing of the core of the thread and the sheath will primarily improve visibility and in particular handling of the thread for a surgeon. In other words, by this means thread characteristics in relation to practical aspects may be improved.

In a preferred embodiment, the surgical thread, in particular the polymeric core and/or the polymeric sheath, may have an additivation. Depending on the type of additive, merely the polymeric core of the thread may have additives, what is preferred according to the invention. This is particularly advantageous, if the barb cut depth at least corresponds to the thickness of the polymeric sheath. In this case, release of additives to the surrounding tissue may be effected at target sites of the thread, where the polymeric core of the thread is exposed due to the cut in barbs. Appropriate additives are in particular biological, medical and/or pharmaceutical agents. Preferred additives are thus selected from the group consisting of antimicrobial, in particular antibiotic, disinfecting, growth-promoting, anti-inflammatory, analgesic and/or odor-controlling active agents. Particularly preferred are growth factors, differentiation factors, recruiting factors and/or adhesion factors. Examples of appropriate growth factors may be selected from the group consisting of fibroblast growth factor (FGF), transforming growth factor (TGF), platelet derived growth factor (PDGF), epidermal growth factor (EGF), granulocyte-macrophage colony stimulation factor (GMCSF), vascular endothelial growth factor (VEGF), insuline-like growth factors (IGF), hepatocyte growth factor (HGF), interleucin-1 B (IL-1 B), interleucin-8 (IL-8), nerve growth factor (NGF), and combinations thereof.

In an advanced embodiment, the additives may be cells, like fibroblasts and/or chondrocytes and/or precursor cells, in particular stem cells, for example. In other words, the thread, in particular the polymeric core and/or the polymeric sheath may be inoculated with cells, particularly body cells, preferably autologous body cells. A thread inoculated with chondrocytes may be applied as meniscus, tendon and/or ligament suture, for example. In other words, the thread is utile in the treatment of meniscus, tendon and/or ligament disorders. Generally, an addition of cells to the thread is beneficial in that substances produced and secreted by the cells will be an active aid in wound healing. Cellular formed collagen may contribute to smoothing of wrinkles, in particular with surgical threads used in plastic surgery, for example. A cellular colonization of the thread will be enhanced in particular as a result of the barbs enlarging and preferably roughening the surface of the thread.

The thread of the present invention preferably has an elongation at break between 15 and 100%, in particular 30 and 90%. It is particularly preferable when the thread of the present invention has a linear tensile strength between 100 and 700 N/mm², preferably 150 and 700 N/mm², in particular 200 and 600 N/mm², based on a thread diameter without barbs.

In a further embodiment, the proportion of the overall volume of the thread which is accounted for by the polymeric sheath is between 34% and 90% by volume, in particular 55% and 84% by volume. Correspondingly, the proportion of the overall volume of the thread which is accounted for by the polymeric core may be between 66% and 10% by volume, in particular 45% and 16% by volume. The surgical thread may also have a thickness between 0.2 and 1.2 mm, in particular 0.25 and 0.9 mm.

The barbs themselves may in principle be configured in different shapes and geometries. For example, the barbs may have an escutcheon, shield, scale, wedge, spike, dart, V and/or W shape. Preferably, the barbs are pointed/sharp at their distal end.

The barbs may further be configured in principle in different arrangements on the surgical thread. For example, the barbs may have a row-shaped arrangement, an offset arrangement, a zigzag-shaped arrangement, an overlapping arrangement, an offset and partially overlapping arrangement, a spiral or helical arrangement, a random arrangement, or combinations thereof, in the longitudinal and/or transverse direction, preferably in the longitudinal direction, of the thread. Particular preference is given to an arrangement in which the barbs are distributed over the entire surface of the thread, since the thread is in this case particularly strongly anchorable in a biological tissue. Particularly preferred is a spiral or helical arrangement of the barbs on the surgical thread. Furthermore, particular emphasis is given to an offset arrangement of the barbs, wherein the barbs are partially overlapping one another. Such an arrangement may be realized for example by forming barbs with a small angular offset and in small intervals between each other on the thread, preferably by shallow cuts into the thread. In such an arrangement, two adjacent barbs form a barb having a twin-tip configuration ("double-acting" barb). Such a twin-tip configuration is primarily advantageous in relation to solid and secure anchoring of the thread in biological tissue.

In a further embodiment, the surgical thread includes at least one set, in particular two, three or more sets, of barbs. A set of barbs is herein to be understood as referring to an arrangement of barbs on the surgical thread that is congruent in respect of the configuration of the barbs, in particular in respect of barb height, barb length, barb cut depth, tip angle, deploy angle, orientation of the barbs and/or form or shape of the barbs.

It is particularly preferable for the surgical thread to have a so-called bidirectional arrangement of barbs. A bidirectional arrangement of barbs is herein to be understood as referring to an arrangement in which the barbs are orientated in two different directions. Preferably, when viewed in the longitudinal direction of the surgical thread, the barbs are formed in a first portion of the thread to face in the direction of a remaining second portion of the thread and in the remaining second portion of the thread to face in the direction of the first portion of the thread. Particularly preferably, when viewed in the longitudinal direction of the surgical thread, the barbs are formed in a first portion of the thread to face the thread midpoint direction and in a remaining second portion of the thread similarly to face in the direction of the thread midpoint. The length of the portions of thread is preferably approximately equal to half the thread length, so that the thread midpoint forms a kind of centre of symmetry. In this way, the surgical thread can be pulled at one end through a biological tissue without major resistance up to about the length midpoint of the thread, the barbs deploying when the thread is pulled in the opposite direction and thereby anchor/retain the thread in the tissue without any need for knotting.

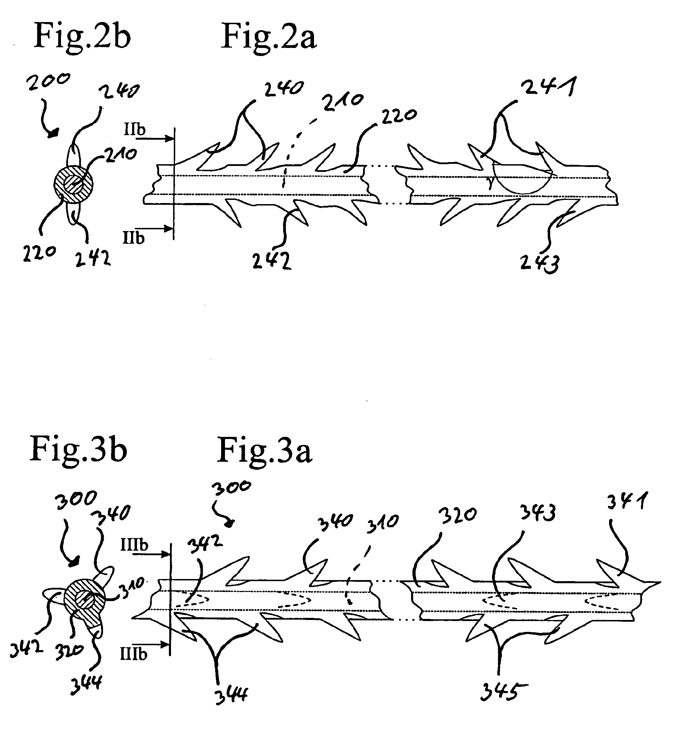

In a particularly advantageous embodiment, the surgical thread has at least two bidirectional arrangements of barbs on its surface. It is particularly preferable when a first bidirectional arrangement of barbs is formed on the thread surface about 180 degrees, in the circumferential direction of the thread, from and preferably offset with respect to a second bidirectional arrangement of barbs on the thread surface (cf. Figures 2a,b). It can further be provided according to the present invention that the surgical thread includes altogether three bidirectional arrangements of barbs. In this case, it is preferable when a first bidirectional arrangement of barbs is formed about 120 degrees, in the circumferential direction of the thread, from and preferably offset with respect to a second bidirectional arrangement of barbs which in turn is formed about 120 degrees, in the circumferential direction of the thread, from and preferably offset with respect to a third bidirectional arrangement of barbs, so that the third bidirectional arrangement of barbs is likewise disposed about 120 degrees, in the circumferential direction of the thread, from and preferably offset with respect to the first bidirectional arrangement of barbs (cf. Figures 3a,b).

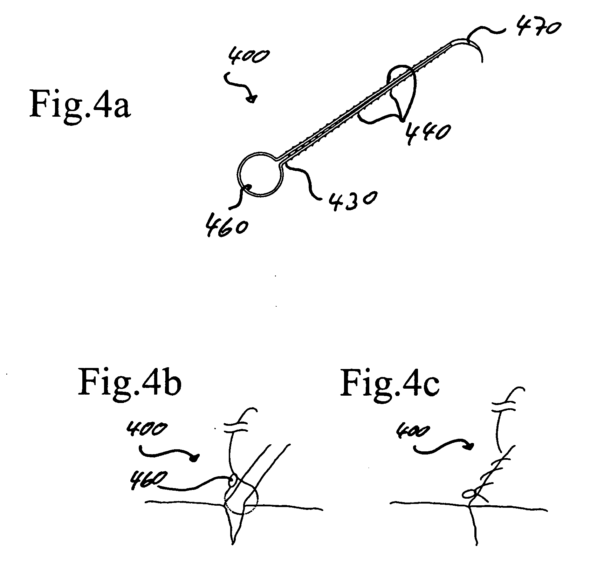

It can further be provided according to the present invention that the surgical thread includes areal regions or portions without barbs. Preferably, the surgical thread has approximately in the region of the thread midpoint an areal portion without barbs. This areal portion may, when considered in the longitudinal direction of the suture, have a length between 0.5 and 5 cm, in particular 1.5 and 3 cm, preferably a length of about 2 cm. As a result, the thread ends can form a loop to lie side by side and preferably be attached to a surgical needle (cf. Figures 4a-c). Preferably, the remaining areal portions of the thread in this embodiment have a bidirectional arrangement of barbs, so that, after forming the loop, the barbs point unidirectionally in the direction of the loop. With regard to possible arrangements for the barbs, reference is made to the preceding description.

In a further-reaching embodiment, the barbs have a so-called apex angle α between 13 and 60 degrees, in particular 15 and 40 degrees. It is particularly preferable according to the present invention when the barbs project from the surface of the surgical thread. Preferably, the barbs have a deploy angle β, measured from the cut surface of the barb underside to the cut surface of the thread body or strand, between 12 and 50 degrees, in particular 17 and 45 degrees. The surgical thread can be characterized by a so-called cut area angle γ which is measured from the cut area of the thread strand to the uncut surface of the thread strand. This cut area angle γ can be between 112 and 167 degrees, in particular 120 and 165 degrees.

The barbs, particularly barbs projecting in the longitudinal direction of the thread, can have a mutual spacing between 0.5 and 5 mm, preferably 1.0 and 2.0 mm, measured from barb tip to barb tip.

In general, the surgical thread is in a drawn state. However, it is preferable according to the present invention when the barbs themselves are formed by cuts into the surgical thread in its undrawn state. Because the thread generally decreases in diameter in any subsequent drawing operation, whereas the barbs essentially retain their original shape and size, completely novel, variable barb geometries can be realized. More particularly, the barbs can have mechanical properties, particularly in relation to hardness, flexibility, bendability and elasticity, which differ from the rest of the thread, the so-called thread main body. This makes it possible for the properties of the barbs to be adapted to the properties of the thread, particularly the polymeric core of the thread, in a controlled manner. It is further of advantage when, as a consequence of a drawing operation being carried out on the thread which has been subjected to a cutting action in the undrawn state, portions of the thread which have been subject to cutting and portions of the thread which have not been subject to cutting become more alike with regard to their diameter. As a result, the barbs can no longer completely disappear into the undercuts originally formed by cutting into the thread, which results in improved anchoring in biological tissues. A further advantage concerns the manufacturing operation. In general, an undrawn thread is softer and its machining is therefore simpler and creates less wear on the cutting device. An additional advantage is that the barbs usually deploy automatically, i.e. without auxiliary means, as a result of a subsequent drawing operation. In general, the barbs can be made to deploy synchronously with a subsequent drawing operation.

In a preferred embodiment, the surgical thread has a circular cross section. The thread preferably has a circular cross section, wherein the proportion of the radius which is accounted for by the polymeric core is preferably between 30 and 90%, in particular 40 and 70%. However, other cross-sectional shapes are also conceivable in principle. For example, the thread may have an oval, triangular or trilobal, square, trapezoidal, rhomboidal, pentagonal, hexagonal, star-shaped or cruciform cross section. Such cross-sectional shapes can readily be realized with the aid of corresponding extrusion dies which can be custom made with any desired cross-sectional shape.

In a further embodiment the thread may have a profiled or moulded surface, in particular a sinuous or wavelike surface. This may be beneficial regarding the formation of barbs with different structures.

The surgical thread preferably comprises a monofil thread, a monofilament. However, it is also possible in principle for the surgical thread to be a multifilament, in particular a multifilament yarn. It can further also be contemplated that the surgical thread is present as a so-called pseudo-monoflament. A pseudomonofilament for the purposes of the present invention is a surgical thread having a sheath-core construction wherein the core is formed of a multifilament.

In a further embodiment, the surgical thread is pointed/sharpened at one end at least, preferably at both ends, in order that its penetration through a biological tissue may be facilitated.

In a further embodiment, at least one end of the thread is attached to a surgical needle. In the case of the loop-shaped thread already described, generally both ends of the thread are attached to one surgical needle. If, by contrast, the thread has a bidirectional arrangement, it is preferable according to the present invention when the two ends of the thread are each attached to a surgical needle. To attach the thread to the surgical needle, the thread is very generally threaded into a hole in the needle and the needle is subsequently crimped together in the region of the hole.

To avoid puncture channel haemorrhages, it can be provided according to the present invention that the thread has a smaller diameter in the region of its ends than in its remaining regions. In other words, the ends of the thread can have a tapered diameter. It is a particular advantage that such a thread can be combined with a surgical needle which is actually designed for smaller thread diameters. In this way it is possible to achieve an alignment of the thread diameter with the needle diameter. The present invention makes it possible to provide a diameter ratio of needle to thread <2:1, preferably 1:1. As a result, the puncture channel formed by the needle is more fully occupied by the thread regions having the original (untapered) diameter. Preferably, the tapered diameter in the region of the thread ends is equal to the diameter of the polymeric core of the thread. The other regions of the thread preferably have the original diameter (including the thickness of the polymeric sheath). Particular preference is given to a thread according to the present invention whose polymeric core has a diameter which is equal to the diameter of a needle hole and the overall diameter of which (including the thickness of the polymeric sheath) is equal to the needle diameter. To taper the diameter, the thread can be peeled off in the region of its ends. Preferably, it is only the polymeric sheath which is peeled off in the region of the thread ends. To peel the thread, thermal methods can be used, particularly laser techniques. The transition from the original diameter of the thread to the tapered diameter in the region of the thread ends can be formed to be abrupt or continuous, particularly in the form of a gradient. To form a gradual transition, extrusion technology is suitable in particular. The withdrawal speed in extruding a thread can be varied, periodically in particular. This can be accomplished for example by modulating the rotary speed of the thread-withdrawing godet. Alternatively, additional godets can be disposed between the extrusion die and the withdrawal godet.

It is particularly preferable according to the present invention when the surgical thread is a surgical suture.

A further aspect of the present invention relates to a surgical kit/set comprising at least a surgical needle and the thread of the present invention. With regard to further features and details relating to the kit/set, reference is made to the preceding description.

The present invention further provides a method of forming the thread of the present invention, wherein a thread core polymer and a sheathing polymer are formed into a thread having a polymeric sheath-core construction and barbs are then cut into the sheath of the thread.

In a preferred embodiment, the thread having the polymeric sheath-core construction is formed by coextrusion, in particular bicomponent extrusion, of the thread core polymer and of the sheathing polymer. In an alternative embodiment, the thread having the polymeric sheath-core construction is formed by a sheathing extrusion, wherein the thread core polymer is sheathed by the sheathing polymer. In this embodiment, the thread core polymer can be used, for example, as monofilament or multifilament, in particular multifilament yarn, so that sheathing coextrusion can also be used to produce pseudomonofilaments, into the sheath of which barbs are subsequently cut.

The barbs are preferably cut into the sheath to a depth (measured perpendicularly from the thread surface) equal to not more than the thickness, in particular exactly the thickness, of the sheath. The barbs are more preferably cut into the sheath to a depth between 10 and 35%, in particular 15 and 30%, of the diameter of the thread.

The barbs may in principle be cut in the drawn or undrawn state of the thread. According to the present invention, it may be contemplated in particular that the barbs are cut into the thread in the undrawn state of the thread and the thread is drawn thereafter. When the barbs are cut in the undrawn state, the barbs may be cut into the undrawn thread in a cut angle θ, relative to the outside surface of the undrawn thread, between 15 and 50 degrees, in particular 20 and 40 degrees. It has emerged that, surprisingly, barbs produced by small cut angles θ can be made to deploy more uprightly by a subsequent drawing of the thread than barbs produced by large cut angles θ. More particularly, the difference between the deploy angle β and the cut angle θ (β-θ) is greater for small cut angles θ than for large cut angles θ. It has further emerged that barbs produced by comparatively large cut angles θ, in particular by cut angles θ ≥ 30 degrees, have more built-up backs. The barb backs in this case generally display a build-up of material, in particular in the form of an accumulation or thickening. This enhances the stiffness, particularly the flexural stiffness, of the hooks, which enhances the anchoring of the thread of the present invention in biological tissues and hence generally the security of wound closure. In other words, choice of the cut angle θ makes it possible to influence barb geometry and create whichever is the best barb geometry for a particular application.

In a further embodiment, the barbs are cut into the thread while the thread is rotated. In accordance with the above explanations, the thread to be treated may be in a drawn or in an undrawn condition. In another embodiment, the barbs are cut into an undrawn thread, and in a subsequent drawing operation the thread may be twisted simultaneously. In another alternative embodiment, the thread may be twisted prior to barb cutting, and again detwisted when the cutting in of barbs is completed. The embodiments specified in this paragraph are particularly advantageous to produce a radial, in particular spiral or helical, arrangement of barbs on the thread surface.

The thread is produced by utilizing a thread core polymer whose melting point is higher, preferably at least 20°C higher, than the melting point of the sheathing polymer.

In a particularly preferred embodiment, the barbs are cut into the sheath thermally, in particular in a temperature range below the melting point of the thread core polymer. This contributes to enhanced cut consistency when producing the barbs. Thermal cutting of barbs further has the advantage over purely mechanical cutting, which is similarly possible according to the present invention, that the cut ends in the thread strand which are produced by thermal cutting are less tapered, more particularly less acute, than result from purely mechanical cutting. This can be used to minimize the risk of the thread developing under load a tear starting from the respective cut ends. Thermal cutting of barbs can be performed for example with the aid of a cutting wire, more particularly a metal wire, suitable for this purpose. Preference is given to using a heated, particularly an electrically heated, cutting wire. The cutting wire may comprise a fine wire. Preference is given to using a cutting wire having a diameter between 20 and 50 µm. As an alternative to a single cutting wire, it is also possible to use a sheet of cutting wires. It is similarly possible to use a metal grid.

In a further suitable embodiment, the barbs are cut into the sheath mechanically, preferably by means of at least one cutting blade. Customary cutting devices can be utilized in this embodiment. These customarily include a cutting bed, at least one cutting blade and also holding or retaining elements, for example a vice, chucks, holding or clamping jaws, and the like. The mechanical cutting of barbs particularly preferably utilizes a cutting bed with a groove, the groove being intended to receive the thread - relaxed or twisted - to be cut into. Depending on the depth of the groove, the use of at least one cutting blade allows specific control of the cut depth to which the barbs are cut into the sheath of the thread. This is because the at least one cutting blade is generally configured such that with it only at most the regions of the thread which protrude from the groove can be cut into. This permits improved cutting consistency when producing barbs.

Laser cutting processes are another way of cutting barbs into the sheath. In other words, the barbs can also be cut into the sheath by means of a laser. Useful lasers include in principle not only gas lasers, for example CO₂ lasers, but also solid state lasers, for example Nd:YAG lasers. In general, a suitable laser cutting machine consists of a laser beam source, a beam guide and a usually mobile system of focusing optics (concave mirror or position lens). The beam leaving the beam source is guided either through an optical fibre in the case of an Nd:YAG laser for example, or via a deflecting mirror, in the case of the CO₂ laser for example, to the machining optics which focus the laser beam and thereby produce the power densities required for cutting, which generally range from 10⁶ to 10⁹ W/cm². Appropriate laser cutting processes are well known to a person skilled in the art, so that more far-reaching observations can be dispensed with here.

When the barbs are produced by cutting into the sheath of an undrawn thread, the thread is very generally drawn subsequently. Drawing is preferably effected by heating, in particular in a temperature range between 20 and 80°C above the glass transition point of the thread. Infrared rays for example can be used to produce heat. Drawing can further be carried out in an oven or with the aid of heatable rolls, rollers or godets. Drawing can be carried out continuously or discontinuously. In continuous drawing, the thread is generally guided over a roller or godet system comprising a set of rollers or godets which can have different speeds of rotation. Usually, each subsequent roller has a higher speed of rotation than the preceding roller of the drawing system. In the case of discontinuous drawing, by contrast, the thread is generally clamped between suitable holding or retaining elements, for example clamping jaws, of a tensioning device and subsequently drawn. To draw the thread it is possible to use a draw ratio between 2.5 and 8, in particular 3 and 5.

After drawing, the surgical thread can be subjected to various post-treatment steps. In general, the thread is for this heat-conditioned in vacuo. This can be used to increase the crystallinity of the thread and reduce its residual monomer content. A further advantage which can be achieved through a post-treatment of the thread relates to the reduced susceptibility to shrinking.

The present invention further provides for the use of the surgical thread as surgical suture, particularly as knotless or self-retaining suture. As already mentioned, the barbs serve to anchor the thread in biological, particularly human and/or animal, tissues. The tissues may comprise for example skin, fat, fascia, bone, muscle, organs, nerves, blood vessels, connective tissue, sinews, tendons or ligaments. The surgical thread is preferably employed in plastic surgery, preferably for tightening the skin. For example, the thread of the present invention is suitable for eyebrow lifts. In addition, however, the thread of the present invention is also suitable for other surgical indications, particularly for indications in which the use of conventional sutures is made difficult by steric hindrance. For example, the surgical thread can be used in laparoscopic interventions, particularly for retaining meshes, for example hernia meshes, prolapse meshes or urinary incontinence meshes. A further possible area of use relates to the performance of anastomoses, in particular vascular or intestinal anastomoses.

Altogether, the thread of the present invention makes it possible to achieve the following advantages in particular:

The sheath-core construction which the present invention provides for the surgical thread makes it possible to choose the polymeric materials for the thread core and the sheath independently of each other. This makes it possible, for example, to use a polymeric material having a low flexural modulus for the thread core and a polymeric material having a comparatively high flexural modulus for the sheath. A thread resulting therefrom has comparatively stiff barbs, relative to its core, without the thread becoming altogether too stiff. This is particularly effectively implementable with threads whose polymeric sheath is of low thickness relative to the polymeric core. Improved stiffness for the barbs can additionally or alternatively be achieved by cutting the barbs in the undrawn state of the thread, particularly at cut angles θ ≥ 30 degrees. High stiffness, in particular flexural stiffness, on the part of the barbs provides a general improvement in the anchoring in biological tissues of the thread of the present invention ("Rawlplug effect") and hence also increases wound closure security. While conventional barbed sutures are in principle prone to the risk of tearing from the respective cut ends, the thread of the present invention generally does not give rise to any tearing beyond the boundary between the core and the sheath. When, more particularly, the polymer chosen for the sheath has a lower melting point than the melting point of the polymer for the polymeric core of the thread, barbs can be cut thermally into the thread without injuring or damaging the core of the thread in the process. This, as already mentioned, contributes to enhanced cutting consistency when producing the barbs on the thread.

Further features of the invention will become apparent from the following description of preferred embodiments with reference to examples and figure descriptions in conjunction with features of the dependent claims and the drawings. Individual features can be actualized either singly or pluraly in combination with each other.

In the schematic drawings:
- Figure 1a: shows an oblique view of an embodiment of the thread of the present invention,
- Figure 1b: shows a surgical thread cut in the undrawn state,
- Figure 1c: shows an embodiment of the thread of the present invention,
- Figure 1d: shows a further embodiment of the thread of the present invention,
- Figure 1e: shows a further embodiment of the thread of the present invention,
- Figure 2a: shows a further embodiment of the thread of the present invention,
- Figure 2b: shows a view of the cross-sectional surface of an embodiment of the thread of the present invention,
- Figure 3a: shows a further embodiment of the thread of the present invention,
- Figure 3b: shows a view of a cross-sectional area of an embodiment of the present invention,
- Figure 4a: shows a further embodiment of the thread of the present invention in combination with a surgical needle,
- Figures 4b,c: show an operating technique using the combination, shown in Figure 4a, of a suture of the present invention and a surgical needle.

### Examples

### Example 1: Producing a surgical thread having a sheath-core construction

A surgical thread having a sheath-core construction was produced using Monosyn® (triblock terpolymer of glycolide, trimethylene carbonate and ε-caprolactone) as thread core polymer and poly(para-dioxanone) (PDO) as sheathing polymer in a volume ratio of 36:64. Production took place with the aid of a bicomponent monofilament range consisting of a twinscrew extruder, a single-screw extruder and also a bicomponent spinning head. The processing conditions employed were as follows:

| | Extruder 1 (Monosyn) | Extruder 2 (PDO) |
|---|---|---|
| Zone 1-3 temperature [°C] | 190/205/215 | 140/160/160 |
| Melt line temperature [°C] | 215 | 180 |
| Spinning pump [ccm/rev.] | 0.25 | 0.25 |
| Spinning pump speed [rpm] | 12.2 | 21.8 |
| Spinning head temperature [°C] | 210 | |
| Die diameter [mm] | 2.0 | |
| Quench bath temperature [°C] | 20 | |
| Air gap [cm] | 3 | |
| Withdrawal speed [m/min] | 5 | |
| Monofilament diameter [mm] | 1.32 | |
| Sheath thickness [mm] | 0.24 | |

The monofilament was subsequently drawn in two stages using the following process parameters:

| | |
|---|---|
| Septett 1 delivery speed [m/min]: | 5.0 |
| Through oven 1 temperature [°C]: | 30 |
| Septett 2 [m/min]: | 22.5 |
| Through oven 2 temperature [°C]: | 130 |
| Septett 3 [m/min]: | 24.3 |
| Overall draw ratio: | 4.86 |

After drawing, the monofilament had an overall diameter of about 0.60 mm and a sheath layer thickness of about 0.11 mm. Linear tensile strength was about 123 N with elongation at break being 38.7%.

### Example 2: Producing a sheath-core bicomponent monofilament with Monosyn® as core polymer and poly(para-dioxanone) (PDO) as sheath polymer in a volume ratio of 16:84

Example 1 was repeated except that the spinning pump speed was 5.4 rpm for extruder 1 (Monosyn®) and 28.6 rpm for extruder 2 (PDO). Monofilament diameter in the undrawn state was again about 1.32 mm, but sheath layer thickness was 0.40 mm. After drawing of the monofilament (same conditions as in Example 1), the overall diameter decreased to 0.60 mm and sheath layer thickness was 0.18 mm. Linear tensile strength was 117 N combined with elongation at break being 42.0%.

### Example 3: Mechanical cutting of barbs into bicomponent monofilaments

The bicomponent monofilaments produced and drawn as per Examples 1 and 2 were placed in grooves in a metal plate which were 0.5 mm (monofilament produced as per Example 1) and 0.43 mm (monofilament produced as per Example 2) deep and 0.65 mm wide, and clamped tight. A microtome was used to cut into the monofilament along an inclined guide plane at an angle of 25 degrees between the blade and the monofilament surface, until the microtome came to rest on the metal plate. This produced cut depths (measured perpendicularly to the monofilament surface) of 0.10 mm (in the case of the monofilament produced as per Example 1) and 0.17 mm (in the case of the monofilament produced as per Example 2). Cut lengths were 0.24 mm (in the case of the monofilament produced as per Example 1) and 0.40 mm (in the case of the monofilament produced as per Example 2). With both monofilaments, the cuts were each limited to the sheath material. The cuts were spaced 0.8 mm apart.

### Example 4: Thermal cutting of barbs into a bicomponent monofilament

The monofilament produced and drawn as per Example 2 was placed in a 0.35 mm deep and 0.65 mm wide groove in a Teflon plate and clamped tight. A fine wire about 35 µm in diameter was clamped into the insulated terminals of a fork-shaped device equipped with a handle. The fine wire was electrically connected to a controllable transformer, such that it could heat up as a function of the applied voltage. Preliminary tests showed that a voltage of about 5 volts was sufficient to heat the fine wire to a temperature sufficient to melt PDO but not Monosyn®.

The heated fine wire was then guided via a device to cut into the monofilament, and after the maximum depth of cut was reached back out again, at an angle of about 25 degrees. Although the monofilament protruded out of the groove by more than the thickness of the sheath layer, the core remained uncut. Further cuts were applied at a spacing of about 1 mm.

### Example 5: Comparative example for mechanical cutting of barbs into a drawn one-component PDO monofilament

A drawn PDO monofilament having a diameter of 0.60 mm was cut as described under Example 3 in grooves 0.50 mm and 0.43 mm deep to form barbs. The barbs had comparable geometries to the barbs produced under Example 3. However, the present monofilament proved to have a distinctly higher flexural stiffness than the monofilaments described in Example 3.

### Example 6: Comparative example for mechanical cutting of barbs into an undrawn one-component PDO monofilament with subsequent drawing

An undrawn PDO monofilament having a diameter of 1.30 mm was placed, one hour after extrusion, into a groove 1.1 mm deep, clamped tight and cut as described under Example 7. Subsequently an attempt was made to draw the monofilament in a batch operation at room temperature. This was unsuccessful, however, since the monofilament would tear, the tears starting from the cuts, before any significant drawing had taken place. Regular drawing was only possible at a drawing temperature of about 48°C. The barbed monofilament obtained in this way proved to have a distinctly higher flexural stiffness than the bicomponent types of Example 7.

### Example 7: Mechanical cutting of barbs into undrawn bicomponent monofilaments with subsequent drawing

Undrawn monofilaments produced as per Examples 1 and 2 were each aged for 30 minutes. This reduced the tackiness of the previously amorphous PDO sheath and its crystallinity increased.

Monofilament pieces 30 cm in length were then placed in a groove having a depth of 1.1 mm (Example 1) or 0.95 mm (Example 2) and a width of 1.35 mm, clamped tight and cut as described under Example 3. The cut angle was about 30 degrees. Further cuts into the monofilament pieces were made at a spacing of 0.5 mm.

This was followed by batchwise drawing in a first step at room temperature. Unlike Example 6, thread rupture did not occur through tearing starting from the respective cut ends. Drawing at 48°C was also possible. In a second step, setting was carried out at 90°C in a circulating air oven in the clamped state. In contrast to cutting in the drawn state, the barbs deployed to higher elevation as a result of this process.

### Example 8: Producing a bicomponent monofilament have a sheath-core construction using polypropylene (PP) as core polymer and polyethylene terephthalate (PET) as sheathing polymer in a volume ratio of 45:55 The materials used were polypropylene having an MFI of 2.8 and polylethylene terephthalate having an intrinsic viscosity of 0.9 dl/g.

| | Extruder 1 (PP) | Extruder 2 (PET) |
|---|---|---|
| Zone 1-3 temperature [°C] | 230/230/230 | 260/280/280 |
| Melt line temperature [°C] | 240 | 270 |
| Spinning pump [ccm/rev.] | 0.25 | 0.25 |
| Spinning pump speed [rpm) | 15.3 | 18.7 |
| Spinning head temperature [°C] | 270 | |
| Die diameter [mm] | 2.0 | |
| Quench bath temperature [°C] | 20 | |
| Air gap [cm] | 3 | |
| Withdrawal speed [m/min] | 5 | |
| Monofilament diameter [mm] | 1.30 | |
| Sheath thickness (mm) | 0.22 | |

The monofilament was subsequently drawn in two stages using the following process parameters:

| | |
|---|---|
| Septett 1 delivery speed [m/min]: | 5.0 |
| Through oven 1 temperature [°C]: | 80 |
| Septett 2 [m/min]: | 25.0 |
| Through oven 2 temperature [°C]: | 140 |
| Septett 3 [m/min]: | 26.0 |
| Overall draw ratio: | 5.2 |

After drawing, the monofilament had an overall diameter of about 0.57 mm and a sheath layer thickness of about 0.1 mm. Linear tensile strength was about 121 N with elongation at break being 35.3%.

### Example 9: Producing a bicomponent monofilament having a sheath-core construction using polyethylene terephthalate (PET) as core polymer and polypropylene (PP) as sheathing polymer in a volume ratio of 16:84

The bicomponent monofilament was produced in essentially the same way as in the process described in Example 8, except that the spinning pump speed was 5.4 rpm for extruder 1 (PP) and 28.6 rpm for extruder 2 (PET). Monofilament diameter in the undrawn state was 1.30 mm, the layer thickness of the polypropylene sheath was 0.39 mm. After drawing, carried out under the conditions recited in Example 8, the overall diameter decreased to 0.57 mm. Sheath layer thickness after drawing was 0.17 mm. Linear tensile strength was found to be 128 N with elongation at break equal to 29.7%.

### Example 10: Mechanical cutting of barbs into an undrawn bicomponent monofilament

An undrawn bicomponent monofilament produced as per Example 8 was placed into a 1.1 mm deep and 1.35 mm wide groove in a metal plate and clamped tight. Thereafter, a microtome was used along an inclined guide plane to cut into the monofilament at an angle of 30 degrees between the blade and the monofilament surface until the microtome came to rest on the metal plate. This resulted in a cut depth, measured perpendicularly to the monofilament surface, of 0.20 mm, which was restricted to the sheath material.

The monofilament was then batch drawn on a hot metal rail at 80°C, causing the barbs to deploy to a distinctly upright position. The barbs proved to be very stiff and pierced effectively into biological tissue without buckling. The monofilament itself turned out to be distinctly slacker in flexural stiffness than a PET one-component monofilament of the same gauge.

### Example 11: Thermal cutting of barbs into a monofilament produced as per Example 9

A monofilament produced as per Example 9 was placed into a 0.35 mm deep and 0.65 mm wide groove in a Teflon plate and subsequently clamped tight. To produce the barbs, a fine wire 35 µm in diameter was clamped into the insulated terminals of a fork-shaped device equipped with a handle. The fine wire was electrically connected to a controllable transformer, such that it could be heated up as a function of the applied voltage. Preliminary tests had found that a voltage of 6.5 volts was sufficient to heat the fine wire to a temperature sufficient to melt polypropylene, but not polyethylene terephthalate.

The monofilament was then cut with the electrically heated fine wire at an angle of 25 degrees via a guiding device. After the maximum depth of cut was reached, the fine wire was guided back out again. The monofilament protruded from the groove to an extent greater than equal to the thickness of the sheath layer. Yet the core of the monofilament remained uncut. Further cuts were applied at a spacing of 1 mm.

### Description of figures

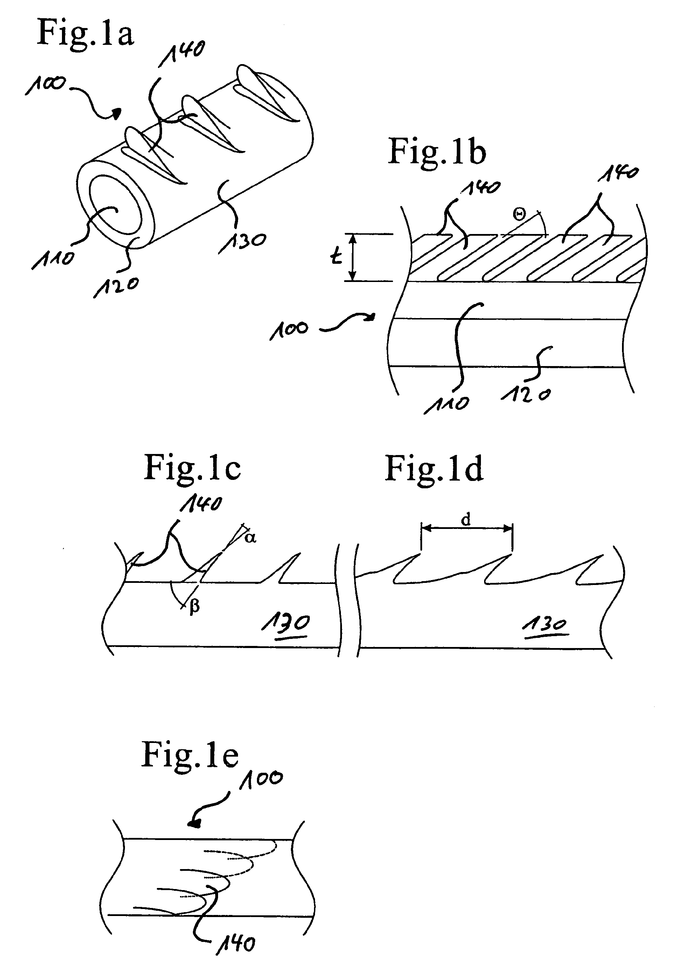

Figure 1a is a schematic view of an inventive thread 100. The thread 100 has a polymeric core 110 and a polymeric sheath 120 surrounding the core 110 (sheath-core construction). The thread 100 further has an elongate main body 130. Barbs 140 have been cut into the polymeric sheath 120 in the longitudinal direction of the thread 100. The polymeric core 110, by contrast, has no cuts.

Figure 1b is a schematic view of the thread 100 of Figure 1a in the undrawn state. The barbs 140 have been produced by cuts into the polymeric sheath 120 of the thread 100. The cuts are made at a cut angle θ to a cut depth t (measured as perpendicular from the thread surface).

Figure 1c is a schematic side view of the thread 100 of Figure 1 a. The barbs 140 are spaced apart by a distance d in the longitudinal direction of the thread 100. The barbs 140 are arranged such that they all face in one direction (unidirectional arrangement). The barbs 140 can be characterized by an apex angle α and a deploy angle β. The apex angle α is to be understood as the angle that results on intersecting an imaginary continuation of the cut surface of the underside of the barb with an imaginary continuation of the back of the barb. The deploy angle β represents the angle that is formed by the cut surfaces of the undersides of the barbs and the corresponding cut surfaces of the elongate main body 130. When the surgical thread 100 is cut in the undrawn state, the barbs 140 can have different geometries depending on the chosen cutting angle θ. Barbs 140 produced by cutting angles θ ≥ 30 degrees for example (cf. Figure 1 d) generally have a more developed back side than barbs 140 produced by smaller cutting angles θ. The built-up configuration of the backs of the barbs is preferably due to a build-up 150 of material in the form of a material accumulation.

Figure 1e is a schematic plan view of another embodiment of the thread 100 according to the present invention. The barbs 140 are formed on the surface of the thread 100 with a small angular offset as well as with small intervals between them. In each case two adjacent barbs 140 together form one barb having a twin-tip configuration ("double-acting" barb). As indicated in figure 1e (dashed barb 140), a spiral-shaped or helical type arrangement of the barbs 140 on the surface of the thread 100 may be produced in this manner.

Figure 2a is a schematic view of an inventive thread 200. The thread 200 has a polymeric core 210 and a polymeric sheath 220 surrounding the core 210 (sheath-core construction). The thread has an elongate main body 230 from which individual barbs 240-243 project. The barbs 240-243 have an offset or staggered bidirectional arrangement on the main body 230. For one half of the thread 200, axially spaced barbs 240 are arranged about 180 degrees, in the circumferential direction, from and offset with respect to the barbs 242. Similarly, for the other half of the thread 200, the barbs 241 are likewise arranged about 180 degrees, in the circumferential direction, from and offset with respect to the barbs 243. The barbs 240 and 241 and the barbs 242 and 243 are each arranged bidirectionally relative to each other. The thread 200 can also be characterized by a cut surface angle γ. The cut surface angle γ is to be understood as the angle measured from the cut surfaces of the elongate main body 230 to the uncut outer surface of the elongate main body 230. Figure 2b is a schematic view of a cross-sectional surface along an imaginary line IIb-IIb of the embodiment described in Figure 2a of an inventive thread 200.

Figure 3a is a schematic view of an inventive thread 300. The thread 300 has a polymeric core 310 and a polymeric sheath 320 surrounding the core 310 (sheath-core construction). The thread 300 further has an elongate main body 330 from which individual barbs 340-345 protrude. The barbs 340-345 have an offset or staggered bidirectional arrangement on the main body 330. For one half of the suture, axially spaced barbs 340 are arranged about 120 degrees, in the circumferential direction, from and offset/stacked with respect to the barbs 342, which in turn are arranged about 120 degrees, in the circumferential direction, from and offset with respect to the axially spaced barbs 344. Consequently, the barbs 344 are similarly arranged about 120 degrees, in the circumferential direction, from and offset with respect to the barbs 340. The same applies mutatis mutandis to the other half of the thread 300 with regard to the barbs 341; 343 and 345. The barbs 340 and 341, the barbs 342 and 343 and the barbs 344 and 345 are each arranged bidirectionally relative to each other.

Figure 3b shows a schematic view of a cross-sectional surface along an imaginary line IIIb-IIIb of the embodiment described in Figure 3a of an inventive thread 300.

Figure 4a is a schematic view of an inventive thread 400, the ends of which lie side by side forming a loop 460 and are attached to a surgical needle 470. There are no barbs in the region of the loop 460, whereas the other regions of the thread 400 have barbs 440 which protrude from a main body 430. The barbs 440 are arranged, in the straightened state of the thread 400 180 degrees, in the circumferential direction of the thread 400, from and offset with respect to each other. After formation of the loop, the barbs 440 face unidirectionally in the direction of the loop 460. The illustrated combination of surgical thread 400 and surgical needle 470 is particularly useful for knotless wound closure. The formation of a loop provides an advantageous way of producing a first secure retention point for closing a wound by threading the thread 400 through the loop 460 (Figure 4b). Starting from this first retention point, a wound is stitched closed with the thread 400, with the barbs 440 anchoring themselves in the tissue region to be closed and thereby constituting additional points of retention (Figure 4c). For clarity, the barbs are not shown in Figures 4b and 4c.

## Claims

1. A surgical thread (100; 200; 300; 400) having a polymeric core (110; 210; 310) and a polymeric sheath (120; 220; 320) surrounding the core (110; 210; 310), wherein the sheath (120; 220; 320) includes barbs (140; 240-243; 340-345; 440) for anchoring in biological, in particular human and/or animal, tissues, **characterized in that** the polymeric core (110; 210; 310) has a higher melting point than the polymeric sheath (120; 220; 320).

2. A surgical thread (100; 200; 300; 400) according to claim 1, **characterized in that** the barbs (140; 240-243; 340-345; 440) have a barb cut depth equal to not more than the thickness, preferably exactly to the thickness, of the polymeric sheath (120; 220; 320).

3. A surgical thread (100; 200; 300; 400) according to claim 1 or 2, **characterized in that** the barbs (140; 240-243; 340-345; 440) have a barb cut depth between 10 and 35%, in particular 15 and 30%, of the diameter of the surgical thread (100; 200; 300; 400).

4. A surgical thread (100; 200; 300; 400) according to any preceding claim, **characterized in that** the polymeric core (110; 210; 310) is configured to be flexible, in particular more flexible than the polymeric sheath (120; 220; 320).

5. A surgical thread (100; 200; 300; 400) according to any preceding claim, **characterized in that** the polymeric core (110; 210; 310) has a lower flexural modulus than the polymeric sheath (120; 220; 320).

6. A surgical thread (100; 200; 300; 400) according to any preceding claim, **characterized in that** the polymeric core (110; 210; 310) has an at least 20°C higher melting point, than the polymeric sheath (120; 220; 320).

7. A surgical thread (100; 200; 300; 400) according to any preceding claim, **characterized in that** the polymeric core (110; 210; 310) is formed of an absorbable polymeric material, preferably having a glass transition point < 30°C.

8. A surgical thread (100; 200; 300; 400) according to any preceding claim, **characterized in that** the polymeric sheath (120; 220; 320) is formed of an absorbable polymeric material, in particular from the group consisting of poly(para-dioxanone), poly(ε-caprolactone), polylactide, polyglycolide, poly(trimethylene carbonate), poly(hydroxybutyric acid), mixtures thereof, copolymers thereof and terpolymers thereof, or is formed of a nonabsorbable polymeric material, in particular from the group consisting of polypropylene, polyethylene terephthalate, polyamide, mixtures thereof and copolymers thereof.

9. A surgical thread (100; 200; 300; 400) according to any preceding claim, **characterized in that** the proportion of the overall volume of the thread (100; 200; 300; 400) which is accounted for by the polymeric sheath (120; 220; 320) is between 34% and 90% by volume, in particular 55% and 84% by volume.

10. A surgical thread (100; 200; 300; 400) according to any preceding claim, **characterized in that** the thread (100; 200; 300; 400) has a circular cross section, wherein the proportion of the radius which is accounted for by the polymeric core (110; 210; 310) is preferably between 30 and 90%, in particular 40 and 70%.

11. A surgical thread (100; 200; 300; 400) according to any preceding claim, **characterized in that** the thread (100; 200; 300; 400) comprises a surgical suture.

12. A surgical thread (100; 200; 300; 400) according to any preceding claim, **characterized in that** the surgical thread is present as a pseudomonofilament.

13. A surgical kit comprising at least one surgical needle (470) and a surgical thread (100; 200; 300; 400) according to any preceding claim.

14. A method of forming a surgical thread (100; 200; 300; 400) wherein a thread core polymer and a sheathing polymer are formed into a thread having a polymeric sheath-core construction and barbs (140; 240-243; 340-345; 440) are then cut into the sheath (120; 220; 320) of the thread, **characterized in that** the thread core polymer used has a higher melting point than the sheathing polymer.

15. A method according to claim 14, **characterized in that** the thread having the polymeric sheath-core construction is formed by sheathing extrusion, wherein the thread core polymer is sheathed by the sheathing polymer.

16. A method according to claims 14 or 15, **characterized in that** the barbs (140; 240-243; 340-345; 440) are cut into the thread in the undrawn state of the thread and the thread is drawn thereafter.

17. A method according to any one of claims 14 to 16, **characterized in that** the thread core polymer used has an at least 20°C higher melting point than the sheathing polymer and wherein the barbs (140; 240-243; 340-345; 440) preferably are cut into the sheath (120; 220; 320) thermally, in particular in a temperature range below the melting point of the thread core polymer.

## Patentansprüche

1. Chirurgischer Faden (100; 200; 300; 400) mit einem Polymerkern (110; 210; 310) und einer Polymerhülle (120; 220; 320), die den Kern (110; 210; 310) umgibt, worin die Hülle (120; 220; 320) Widerhaken (140; 240-243; 340-345; 440) zur Verankerung in biologischen, insbesondere menschlichen und/oder tierischen Geweben aufweist, **dadurch gekennzeichnet, dass** der Polymerkern (110; 210; 310) einen höheren Schmelzpunkt aufweist als die Polymerhülle (120; 220; 320).

2. Chirurgischer Faden (100; 200; 300; 400) nach Anspruch 1, **dadurch gekennzeichnet, dass** die Widerhaken (140; 240-243; 340-345; 440) eine Widerhaken-Schneidetiefe aufweisen, die nicht mehr als der Dicke, vorzugsweise genau der Dicke, der Polymerhülle (120; 220; 320) entspricht.

3. Chirurgischer Faden (100; 200; 300; 400) nach Anspruch 1 oder 2, **dadurch gekennzeichnet, dass** die Widerhaken (140; 240-243; 340-345; 440) eine Widerhaken-Schneidetiefe zwischen 10 und 35%, insbesondere 15 und 30%, des Durchmessers des chirurgischen Fadens (100; 200; 300; 400) aufweisen.

4. Chirurgischer Faden (100; 200; 300; 400) nach einem der vorhergehenden Ansprüche, **dadurch gekennzeichnet, dass** der Polymerkern (110; 210; 310) flexibel, insbesondere flexibler als die Polymerhülle (120; 220; 320), ausgebildet ist.

5. Chirurgischer Faden (100; 200; 300; 400) nach einem der vorhergehenden Ansprüche, **dadurch gekennzeichnet, dass** der Polymerkern (110; 210; 310) ein geringeres Biegemodul aufweist als die Polymerhülle (120; 220; 320).

6. Chirurgischer Faden (100; 200; 300; 400) nach einem der vorhergehenden Ansprüche, **dadurch gekennzeichnet, dass** der Polymerkern (110; 210; 310) einen um zumindest 20°C höheren Schmelzpunkt aufweist als die Polymerhülle (120; 220; 320).

7. Chirurgischer Faden (100; 200; 300; 400) nach einem der vorhergehenden Ansprüche, **dadurch gekennzeichnet, dass** der Polymerkern (110; 210; 310) aus einem resorbierbaren Polymermaterial, vorzugsweise mit einem Glasübergangspunkt von <30°C, geformt ist.

8. Chirurgischer Faden (100; 200; 300; 400) nach einem der vorhergehenden Ansprüche, **dadurch gekennzeichnet, dass** die Polymerhülle (120; 220; 320) aus einem resorbierbaren Polymermaterial, insbesondere aus der aus Poly(para-dioxanon), Poly(ε-caprolacton), Polylactid, Polyglycolid, Poly(trimethylencarbonat), Poly(hydroxybuttersäure), Gemischen davon, Copolymeren davon und Terpolymeren davon bestehenden Gruppe, oder aus einem nicht-resorbierbaren Polymermaterial, insbesondere aus der aus Polypropylen, Polyethylenterephthalat, Polyamid, Gemischen davon und Copolymeren davon bestehenden Gruppe geformt ist.

9. Chirurgischer Faden (100; 200; 300; 400) nach einem der vorhergehenden Ansprüche, **dadurch gekennzeichnet, dass** der Anteil des Gesamtvolumens des Fadens (100; 200; 300; 400), den die Polymerhülle (120; 220; 320) ausmacht, zwischen 34 Volumen-% und 90 Volumen-%, insbesondere zwischen 55 Volumen-% und 84 Volumen-%, beträgt.

10. Chirurgischer Faden (100; 200; 300; 400) nach einem der vorhergehenden Ansprüche, **dadurch gekennzeichnet, dass** der Faden (100; 200; 300; 400) einen kreisförmigen Querschnitt aufweist, worin der Anteil des Radius, den der Polymerkern (110; 210; 310) ausmacht, vorzugsweise zwischen 30 und 90%, insbesondere zwischen 40 und 70% beträgt.

11. Chirurgischer Faden (100; 200; 300; 400) nach einem der vorhergehenden Ansprüche, **dadurch gekennzeichnet, dass** der Faden (100; 200; 300; 400) ein chirurgisches Nahtmaterial umfasst.

12. Chirurgischer Faden (100; 200; 300; 400) nach einem der vorhergehenden Ansprüche, **dadurch gekennzeichnet, dass** der chirurgische Faden als Pseudomonofilament vorliegt.

13. Chirurgisches Kit, das zumindest eine chirurgische Nadel (470) und einen chirurgischen Faden (100; 200; 300; 400) nach einem der vorhergehenden Ansprüche umfasst.

14. Verfahren zur Bildung eines chirurgischen Fadens (100; 200; 300; 400), worin ein Fadenkernpolymer und ein Hüllenpolymer zu einem Faden mit einer Polymer-Hüllen-Kern-Konstruktion geformt werden und Widerhaken (140; 240-243; 340-345; 440) dann in die Hülle (120; 220; 320) des Fadens geschnitten werden, **dadurch gekennzeichnet, dass** das verwendete Fadenkernpolymer einen höheren Schmelzpunkt aufweist als das Hüllenpolymer.

15. Verfahren nach Anspruch 14, **dadurch gekennzeichnet, dass** der Faden mit der Polymer-Hüllen-Kern-Konstruktion durch Hüllenextrusion geformt ist, worin das Fadenkernpolymer mit dem Hüllenpolymer umhüllt wird.

16. Verfahren nach Anspruch 14 oder 15, **dadurch gekennzeichnet, dass** die Widerhaken (140; 240-243; 340-345; 440) im unverstreckten Zustand des Fadens in den Faden geschnitten werden und der Faden anschließend verstreckt wird.

17. Verfahren nach einem der Ansprüche 14 bis 16, **dadurch gekennzeichnet, dass** das verwendete Fadenkernpolymer einen um zumindest 20°C höheren Schmelzpunkt aufweist als das Hüllenpolymer und worin die Widerhaken (140; 240-243; 340-345; 440) vorzugsweise thermisch, insbesondere in einem Temperaturbereich unter dem Schmelzpunkt des Fadenkernpolymers, in die Hülle (120; 220; 320) geschnitten werden.

## Revendications

1. Fil chirurgical (100 ; 200 ; 300 ; 400) comportant une âme polymère (110 ; 210 ; 310) et une gaine polymère (120 ; 220 ; 320) entourant l'âme (110 ; 210 ; 310), la gaine (120 ; 220 ; 320) comprenant des barbillons (140 ; 240-243 ; 340-345 ; 440) pour un ancrage dans des tissus biologiques, en particulier des tissus humains et/ou animaux, **caractérisé en ce que** l'âme polymère (110 ; 210 ; 310) a un point de fusion plus élevé que celui de la gaine polymère (120 ; 220 ; 320).

2. Fil chirurgical (100 ; 200 ; 300 ; 400) selon la revendication 1, **caractérisé en ce que** les barbillons (140 ; 240-243 ; 340-345 ; 440) ont une profondeur d'incision ne dépassant pas l'épaisseur, préférablement correspondant exactement à l'épaisseur, de la gaine polymère (120 ; 220 ; 320).

3. Fil chirurgical (100 ; 200 ; 300 ; 400) selon la revendication 1 ou 2, **caractérisé en ce que** les barbillons (140 ; 240-243 ; 340-345 ; 440) ont une profondeur d'incision représentant de 10 % à 35 %, en particulier de 15 % à 30 %, du diamètre du fil chirurgical (100 ; 200 ; 300 ; 400).

4. Fil chirurgical (100 ; 200 ; 300 ; 400) selon l'une quelconque des revendications précédentes, **caractérisé en ce que** l'âme polymère (110 ; 210 ; 310) est configurée pour être flexible, en particulier plus flexible que la gaine polymère (120 ; 220 ; 320).

5. Fil chirurgical (100 ; 200 ; 300 ; 400) selon l'une quelconque des revendications précédentes, **caractérisé en ce que** l'âme polymère (110 ; 210 ; 310) a un module de flexion inférieur à celui de la gaine polymère (120 ; 220 ; 320).

6. Fil chirurgical (100 ; 200 ; 300 ; 400) selon l'une quelconque des revendications précédentes, **caractérisé en ce que** l'âme polymère (110 ; 210 ; 310) a un point de fusion qui est d'au moins 20 °C supérieur à celui de la gaine polymère (120 ; 220 ; 320).

7. Fil chirurgical (100 ; 200 ; 300 ; 400) selon l'une quelconque des revendications précédentes, **caractérisé en ce que** l'âme polymère (110 ; 210 ; 310) est formée d'un matériau polymère absorbable, ayant préférablement un point de transition vitreuse strictement inférieur à 30 °C.

8. Fil chirurgical (100 ; 200 ; 300 ; 400) selon l'une quelconque des revendications précédentes, **caractérisé en ce que** la gaine polymère (120 ; 220 ; 320) est formée d'un matériau polymère absorbable, en particulier du groupe constitué d'une poly(para-dioxanone), d'une poly(ε-caprolactone), d'un polylactide, d'un polyglycolide, d'un poly(carbonate de triméthylène), d'un poly(acide hydroxybutyrique), de mélanges de ceux-ci, de copolymères de ceux-ci et de terpolymères de ceux-ci, ou est formée d'un matériau polymère non absorbable, en particulier du groupe constitué d'un polypropylène, d'un téréphtalate de polyéthylène, d'un polyamide, de mélanges de ceux-ci et de copolymères de ceux-ci.

9. Fil chirurgical (100 ; 200 ; 300 ; 400) selon l'une quelconque des revendications précédentes, **caractérisé en ce que** la proportion du volume total du fil (100 ; 200 ; 300 ; 400) qui est représentée par la gaine polymère (120 ; 220 ; 320) est de 34 % à 90 % en volume, en particulier de 55 % à 84 % en volume.

10. Fil chirurgical (100 ; 200 ; 300 ; 400) selon l'une quelconque des revendications précédentes, **caractérisé en ce que** le fil (100 ; 200 ; 300 ; 400) a une section transversale circulaire, la proportion du rayon qui est représentée par l'âme polymère (110 ; 210 ; 310) étant préférablement de 30 % à 90 %, en particulier de 40 % à 70 %.

11. Fil chirurgical (100 ; 200 ; 300 ; 400) selon l'une quelconque des revendications précédentes, **caractérisé en ce que** le fil (100 ; 200 ; 300 ; 400) comprend une suture chirurgicale.

12. Fil chirurgical (100 ; 200 ; 300 ; 400) selon l'une quelconque des revendications précédentes, **caractérisé en ce que** le fil chirurgical est présent sous la forme d'un pseudomonofilament.

13. Trousse chirurgicale comprenant au moins une aiguille chirurgicale (470) et un fil chirurgical (100 ; 200 ; 300 ; 400) selon l'une quelconque des revendications précédentes.

14. Procédé de formation d'un fil chirurgical (100 ; 200 ; 300 ; 400), dans lequel un polymère d'âme de fil et un polymère de gaine sont formés pour produire un fil comportant une âme gainée polymère, et des barbillons (140 ; 240-243 ; 340-345 ; 440) sont ensuite entaillés dans la gaine (120 ; 220 ; 320) du fil, **caractérisé en ce que** le polymère d'âme de fil utilisé a un point de fusion plus élevé que celui du polymère de gaine.

15. Procédé selon la revendication 14, **caractérisé en ce que** le fil comportant une âme gainée polymère est formé par extrusion d'une gaine, le polymère d'âme de fil étant gainé par le polymère de gaine.

16. Procédé selon les revendications 14 ou 15, **caractérisé en ce que** les barbillons (140 ; 240-243 ; 340-345 ; 440) sont entaillés dans le fil tandis que le fil est dans l'état non étiré, le fil étant étiré ensuite.

17. Procédé selon l'une quelconque des revendications 14 à 16, **caractérisé en ce que** le polymère d'âme de fil utilisé a un point de fusion qui est d'au moins 20 °C supérieur à celui du polymère de gaine et dans lequel les barbillons (140 ; 240-243 ; 340-345 ; 440) sont préférablement entaillés dans la gaine (120 ; 220 ; 320) par un procédé thermique, en particulier dans une plage de température inférieure au point de fusion du polymère d'âme de fil.
